# EUROPEAN PATENT APPLICATION

(11) **EP 4 417 699 A1**
(43) Date of publication of application: **21.08.2024**
(21) Application number: 22883945.2
(22) Date of filing: 18.10.2022
(51) Int. Cl.: C12P 19/56, C12N 9/10

(54) **METHOD FOR PRODUCING REBAUDIOSIDE D AND REBAUDIOSIDE M**

(30) Priority: 19.10.2021 KR 20210139473
(71) Applicant: CJ CheilJedang Corporation, Seoul 04560 (KR)
(72) Inventor: KIM, Jungeun, Seoul 04560 (KR); PARK, Sunghee, Seoul 04560 (KR); KIM, Tae Kyung, Seoul 04560 (KR); OH, Kyoungmi, Seoul 04560 (KR)
(74) Representative: Mewburn Ellis LLP
(86) International application number: PCT/KR2022/015809
(87) International publication number: WO 2023/068722

(57) **Abstract**

The present application relates to methods for preparing rebaudioside D and rebaudioside M by reaction of uridine diphosphate (UDP)-glycosyltransferases; and compositions for preparing rebaudioside D and rebaudioside M, comprising uridine diphosphate (UDP)-glycosyltransferases.

## Description

### [Technical Field]

The present application relates to methods for preparing rebaudioside D and rebaudioside M by reaction of uridine diphosphate (UDP)-glycosyltransferases; and compositions for preparing rebaudioside D and rebaudioside M, including uridine diphosphate (UDP)-glycosyltransferases.

### [Background Art]

As the World Health Organization (WHO) recommends lowering the amount of daily sugar intake due to concerns about disease (obesity) caused by sugar consumption, various policies aimed at reducing the amount of sugar intake are actively being discussed by the governments of developed countries. Therefore, as the need for developing various alternative sweeteners in place of sugar and high fructose is increasing in the market, alternative sweeteners are continuously being developed and commercialized.

Alternative sweeteners are the subject of continuous variation in the form of synthetic high-intensity sweeteners (e.g., saccharin, aspartame, sucralose, *etc*.), synthetic sugar alcohols (e.g., maltitol and xylitol), and high-intensity sweeteners (e.g., rebaudioside A and liquorice). Nevertheless, due to concerns over the safety of synthetic sweeteners, customers' need for natural sweeteners has been steadily increasing; however, because of limitations to peculiar flavor properties of natural sweeteners (i.e., off-odor and off-flavor), the natural sweeteners cannot fully replace existing low-calorie and zero-calorie products based on synthetic sweeteners.

A natural high-intensity sweetener that has received considerable attention in recent years is stevia extracted from the leaves of Stevia. Stevia has a potential use as an alternative sweetener because it has been reported that it does not generate calories, it is positive for blood glucose and insulin levels, and has no side effects on the human body; however, stevia has a limitation in use for reducing the amount of sugar because it has a bitter taste.

Stevia is a perennial plant in the Asteraceae family native to Paraguay, South America, and its scientific name is *Stevia rebaudiana* Bertoni. The leaves of Stevia contain sweetening components which have 200 to 300 times the sweetness of sugar, and the sweetening components are extracted and used as natural sweetener. The sweetening components of the Stevia extracts contain various steviol glycosides such as stevioside, rebaudioside A, rebaudioside C, rebaudioside D, rebaudioside M, rebaudioside I, and rebaudioside E, *etc.*

The Stevia leaves contain relatively high contents of stevioside (STV), rebaudioside A (Reb A), and rebaudioside C (Reb C) among the sweetening components of the Stevia extract, and thus, high-purity extracted and purified industrial products have been launched, but they have a limitation in use for reducing the amount of sugar because of bitter taste.

Meanwhile, rebaudioside D (Reb D) and rebaudioside M (Reb M) have less bitter taste than STV, rebaudioside A, and rebaudioside C, and have excellent sweetening quality, and thus are highly valuable as alternative sweeteners. However, rebaudioside D and rebaudioside M are present only in very small amounts in the Stevia leaves, so there is a disadvantage in that a method of extracting and purifying rebaudioside D and rebaudioside M from the leaves and producing the same requires a high cost.

### [Prior Art Literature]

### [Patent Literature]

(Patent Literature 0001) Korean Patent No. 1404728

### [Disclosure]

### [Technical Problem]

As a result of intensive efforts to develop an enzyme that has an activity of converting rebaudioside A to rebaudioside D, the present applicant has discovered that the polypeptide sequence whose glycosyltransferase activity was previously unknown has glycosyltransferase activity, and has completed this application by confirming that the polypeptide has glycosyltransferase activity to convert rebaudioside A to rebaudioside D.

### [Technical Solution]

One object of the present application is to provide a method for preparing rebaudioside D, including: reacting nucleotide diphosphate to which glucose is bonded with rebaudioside A in the presence of uridine diphosphate (UDP)-glycosyltransferase B (UGT-B) to prepare rebaudioside D, wherein the uridine diphosphate (UDP)-glycosyltransferase B (UGT-B) is at least one protein selected from the group consisting of proteins consisting of an amino acid sequence of SEQ ID NOS: 1 to 3.

Another object of the present application is to provide a method for preparing rebaudioside M, including: reacting nucleotide diphosphate to which glucose is bonded with rebaudioside A in the presence of uridine diphosphate (UDP)-glycosyltransferase B (UGT-B) to prepare rebaudioside D; and reacting the rebaudioside D with nucleotide diphosphate to which glucose is bonded in the presence of uridine diphosphate (UDP)-glycosyltransferase A (UGT-A) to prepare rebaudioside M, wherein the uridine diphosphate (UDP)-glycosyltransferase B (UGT-B) is at least one protein selected from the group consisting of proteins consisting of an amino acid sequence of SEQ ID NOS: 1 to 3.

Still another object of the present application is to provide a method for preparing rebaudioside D from rebaudioside A, including: reacting sucrose, nucleotide diphosphate, rebaudioside A, sucrose synthase and uridine diphosphate (UDP)-glycosyltransferase B (UGT-B) *in situ* to prepare rebaudioside D.

Yet another object of the present application is to provide a method for preparing rebaudioside M from rebaudioside A, including: reacting sucrose, nucleotide diphosphate, rebaudioside A, rebaudioside D, sucrose synthase, uridine diphosphate (UDP)-glycosyltransferase A (UGT-A) and uridine diphosphate (UDP)-glycosyltransferase B (UGT-B) *in situ* to prepare rebaudioside M.

Even another object of the present application is to provide a composition for preparing rebaudioside D, including uridine diphosphate (UDP)-glycosyltransferase B (UGT-B).

Further another object of the present application is to provide a composition for preparing rebaudioside M, including: uridine diphosphate (UDP)-glycosyltransferase A (UGT-A).

Further another object of the present application is to provide a use of uridine diphosphate (UDP)-glycosyltransferase B (UGT-B) to convert rebaudioside A to rebaudioside D, wherein the uridine diphosphate (UDP)-glycosyltransferase B (UGT-B) is at least one protein selected from the group consisting of proteins consisting of an amino acid sequence of SEQ ID NOS: 1 to 3.

### [Advantageous Effects]

The preparation method of rebaudioside D and rebaudioside M using uridine diphosphate (UDP)-glycosyltransferase B (UGT-B) according to the present application can provide rebaudioside D and rebaudioside M in high purity and high yield, with almost no by-products, and thus can be effectively used for mass production of rebaudioside D and rebaudioside M, because it is economical due to the use of inexpensive raw materials, and the procedure is simple and less time-consuming.

### [Brief Description of Drawings]

Fig. 1 is an HPLC analysis result showing that rebaudioside A is converted to rebaudioside D by uridine diphosphate (UDP)-glycosyltransferase B (UGT-B_6).
Fig. 2 is an HPLC analysis result showing that rebaudioside A is converted to rebaudioside D, rebaudioside D isomer, and rebaudioside M isomer by uridine diphosphate (UDP)-glycosyltransferase B (UGT-B_7).
Fig. 3 is an HPLC analysis result showing that rebaudioside A is converted to rebaudioside D by uridine diphosphate (UDP)-glycosyltransferase B (UGT-B_8).
Fig. 4 is an HPLC analysis result showing that rebaudioside D, rebaudioside M, rebaudioside I, and rebaudioside A are produced from stevioside by uridine diphosphate (UDP)-glycosyltransferase B (UGT-B_6), uridine diphosphate (UDP)-glycosyltransferase A (UGT-A), and sucrose synthase.
Fig. 5 is an HPLC analysis result showing that rebaudioside M isomer, rebaudioside D, rebaudioside M, rebaudioside I, and rebaudioside A are produced from stevioside by uridine diphosphate (UDP)-glycosyltransferase B (UGT-B_7), uridine diphosphate (UDP)-glycosyltransferase A (UGT-A), and sucrose synthase.
Fig. 6 is an HPLC analysis result showing that rebaudioside D, rebaudioside M, rebaudioside I, and rebaudioside A are produced from stevioside by uridine diphosphate (UDP)-glycosyltransferase B (UGT-B_8), uridine diphosphate (UDP)-glycosyltransferase A (UGT-A), and sucrose synthase.
Fig. 7 is an HPLC analysis result showing that rebaudioside D is converted to rebaudioside M by uridine diphosphate (UDP)-glycosyltransferase A (UGT-A).
Fig. 8 is an LC-MS/MS analysis result of rebaudioside D isomer.
Fig. 9 is an LC-MS/MS analysis result of rebaudioside D.
Fig. 10 is an LC-MS/MS analysis result of rebaudioside M isomer.
Fig. 11 is an LC-MS/MS analysis result of rebaudioside M.

### [Detailed Description of Preferred Embodiments]

The present application will be described in detail as follows. Meanwhile, each description and embodiment disclosed herein can be applied to other descriptions and embodiments, respectively. That is, all combinations of various elements disclosed herein fall within the scope of the present application. Further, the scope of the present application is not limited by the specific description described below. Additionally, a number of papers and patent documents have been cited throughout the present specification. The content of the cited papers and patent documents is incorporated herein by reference in their entirety and the level of technical field to which the present application belongs and the contents of the present application will be described more clearly.

One aspect of the present application provides a method for preparing rebaudioside D, including: reacting nucleotide diphosphate to which glucose is bonded with rebaudioside A in the presence of uridine diphosphate (UDP)-glycosyltransferase B (UGT-B) to prepare rebaudioside D, wherein the uridine diphosphate (UDP)-glycosyltransferase B (UGT-B) is at least one protein selected from the group consisting of proteins consisting of an amino acid sequence of SEQ ID NOS: 1 to 3.

As used herein, the term "uridine diphosphate (UDP)-glycosyltransferase (UGT)" is an enzyme that catalyzes the transfer of a monosaccharide moiety from a glycosyl donor to a glycosyl acceptor molecule, and in particular, it refers to an enzyme that utilizes UDP-sugar as the glycosyl donor. In the present application, the UDP-glycosyltransferase may be interchangeably used with UGT.

The UDP-glycosyltransferase may be one produced from recombinant E. *coli, Bacillus,* yeast, *Corynebacterium* or *Agrobacterium* transformed with a vector containing a glycosyltransferase gene, and the UDP-glycosyltransferase may be further purified after production from E. *coli* or the like, or commercially manufactured products may be purchased and used, but the UDP-glycosyltransferase is not limited thereto. In addition, the UDP-glycosyltransferase is known in the art, and the protein and gene sequence of the UDP-glycosyltransferase can be obtained from a known database, for example, GenBank of NCBI, *etc.,* but is not limited thereto.

In the present application, it has been newly discovered that the novel uridine diphosphate (UDP)-glycosyltransferase B (UGT-B), which is at least one protein selected from the group consisting of proteins consisting of an amino acid sequence of SEQ ID NOS: 1 to 3, has an enzyme activity of converting rebaudioside A to rebaudioside D, thereby providing a novel use of UGT-B.

Specifically, the uridine diphosphate (UDP)-glycosyltransferase B (UGT-B) of the present application may have and/or include an amino acid sequence of SEQ ID NO: 1, an amino acid sequence of SEQ ID NO: 2 or an amino acid sequence of SEQ ID NO: 3, or essentially consist or consist of the amino acid sequences.

Additionally, the uridine diphosphate (UDP)-glycosyltransferase B (UGT-B) may include an amino acid sequence having a homology or identity of at least 70%, 75%, 80%, 85%, 90%, 95%, 96%, 97%, 98%, 99%, 99.5%, 99.7%, or 99.9% or more with the amino acid sequence of SEQ ID NO: 1, the amino acid sequence of SEQ ID NO: 2, or the amino acid sequence of SEQ ID NO: 3. In addition, it is apparent that any uridine diphosphate (UDP)-glycosyltransferase B (UGT-B) having an amino acid sequence, in which part of the sequence is deleted, modified, substituted, conservatively substituted, or added, may also fall within the scope of the present application as long as the amino acid sequence has such a homology or identity and exhibits an effect corresponding to that of the uridine diphosphate (UDP)-glycosyltransferase B (UGT-B).

As used herein, although it is described as "a polypeptide or protein including an amino acid sequence described by a specific sequence number", "a polypeptide or protein consisting of an amino acid sequence described by a specific sequence number", or "a polypeptide or protein having an amino acid sequence described by a specific sequence number", it is apparent that any protein having an amino acid sequence in which part of the sequence is deleted, modified, substituted, conservatively substituted or added can be used in the present application even if it has the same or corresponding activity as the polypeptide consisting of the amino acid sequence of the corresponding sequence number. For example, it may be a case where the N-terminus and/or C-terminus of the amino acid sequence is added with a sequence that does not alter the function of the protein, a naturally occurring mutation, a potential mutation thereof (a silent mutation), or a conservative substitution.

For example, it may be a case where the N-terminus, C-terminus and/or inside of the amino acid sequence is added or deleted with a sequence that does not alter the function of the uridine diphosphate (UDP)-glycosyltransferase B (UGT-B) of the present application, a naturally occurring mutation, a potential mutation thereof (a silent mutation), or a conservative substitution.

As used herein, the term "conservative substitution" refers to substitution of an amino acid with another amino acid having similar structural and/or chemical properties. Such amino acid substitution may generally occur based on similarity of polarity, charge, solubility, hydrophobicity, hydrophilicity, and/or amphipathic nature of a residue. For example, positively charged (basic) amino acids include arginine, lysine, and histidine; negatively charged (acidic) amino acids include glutamic acid and aspartic acid; aromatic amino acids include phenylalanine, tryptophan, and tyrosine; and hydrophobic amino acids include alanine, valine, isoleucine, leucine, methionine, phenylalanine, tyrosine, and tryptophan. In addition, amino acids can be classified into amino acids with electrically charged side chains and amino acids with uncharged side chains, and examples of the amino acids with electrically charged side chains include aspartic acid, glutamic acid, lysine, arginine, and histidine. The amino acids with uncharged side chains can be further classified into nonpolar amino acids or polar amino acids. Examples of the non-polar amino acids are glycine, alanine, valine, leucine, isoleucine, methionine, phenylalanine, tryptophan, proline, and examples of the polar amino acids include serine, threonine, cysteine, tyrosine, asparagine, and glutamine. Typically, conservative substitutions have little or no effect on the activity of the resulting polypeptide. Typically, conservative substitutions may have little or no effect on the activity of the protein or polypeptide.

Additionally, the uridine diphosphate (UDP)-glycosyltransferase B (UGT-B) may also include deletion or addition of amino acids that have minimal influence on the properties and secondary structure of a polypeptide. For example, the polypeptide may be conjugated with a signal (or leader) sequence at the N-terminus involved in the transfer of proteins co-translationally or post-translationally. Further, the polypeptide may also be conjugated with another sequence or linker to identify, purify, or synthesize the polypeptide.

As used herein, the term "homology" or "identity" refers to a degree of relatedness between two given amino acid sequences or nucleotide sequences, and may be expressed as a percentage. The terms homology and identity may often be used interchangeably with each other.

The sequence homology or identity of conserved polynucleotide or polypeptide sequences may be determined by standard alignment algorithms and can be used with a default gap penalty established by the program being used. Substantially, homologous or identical sequences are generally expected to hybridize to all or part of the sequences under moderate or high stringent conditions. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

In one example of the present application, the uridine diphosphate (UDP)-glycosyltransferase B (UGT-B) may have an activity of converting rebaudioside A to rebaudioside D.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the "corresponding region" generally refers to a similar or corresponding position in the related protein or reference protein.

For example, any amino acid sequence is aligned with SEQ ID NO: 1, and based on the alignment, each amino acid residue of the amino acid sequence can be numbered with reference to the numerical position of the amino acid residue corresponding to the amino acid residue of SEQ ID NO: 1. For example, a sequence alignment algorithm such as that described herein can identify the position of an amino acid or a position where modifications such as substitutions, insertions or deletions occur compared to a query sequence (also referred to as a "reference sequence").

Example of the alignment may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277), *etc.,* but is not limited thereto, and sequence alignment programs, such as pairwise sequence comparison algorithms, *etc.,* known in the art may be appropriately used.

Additionally, a polynucleotide encoding the uridine diphosphate (UDP)-glycosyltransferase B (UGT-B) of the present application is not only a nucleotide sequence encoding the amino acids represented by each SEQ ID NOS above, but also a nucleotide sequence exhibiting a homology of 80% or more, preferably 90% or more, more preferably 95% or more, even more preferably 98% or more, and most preferably 99% or more with the above sequence, and any gene sequence encoding a protein exhibiting the same or corresponding efficacy as each of the above proteins is included without limitation. In addition, it is apparent that any any nucleotide sequence, in which part of the sequence is deleted, modified, substituted, or added, may also fall within the scope of the present application as long as the nucleotide sequence has such a homology.

As used herein, the term "polynucleotide", which is a polymer of nucleotides composed of nucleotide monomers connected in a lengthy chain by a covalently bond, is a DNA or RNA strand having at least a certain length. More specifically, it may refer to a polynucleotide fragment encoding the uridine diphosphate (UDP)-glycosyltransferase B (UGT-B).

The polynucleotide encoding the uridine diphosphate (UDP)-glycosyltransferase B (UGT-B) of the present application may include a nucleotide sequence encoding the amino acid sequence of SEQ ID NO: 1, SEQ ID NO: 2, or SEQ ID NO: 3. As an example of the present application, the polynucleotide may have or include the sequence of SEQ ID NO: 6, SEQ ID NO: 7, or SEQ ID NO: 8. In addition, the polynucleotide may consist or consist essentially of the sequence of SEQ ID NO: 6, SEQ ID NO: 7, or SEQ ID NO: 8.

The polynucleotide of the present application may undergo various modifications in the coding region within the scope that does not change the amino acid sequence of the uridine diphosphate (UDP)-glycosyltransferase B (UGT-B) of the present application, due to codon degeneracy or in consideration of the codons preferred in an organism in which the uridine diphosphate (UDP)-glycosyltransferase B (UGT-B) of the present application is to be expressed. Specifically, the polynucleotide of the present application may have or include a nucleotide sequence having a homology or identity of 70% or more, 75% or more, 80% or more, 85% or more, 90% or more, 95% or more, 96% or more, 97% or more, 98% or more, and 100% or less with the sequence of SEQ ID NO: 6, SEQ ID NO: 7, or SEQ ID NO: 8, or may consist or consist essentially of a nucleotide sequence having a homology or identity of 70% or more, 75% or more, 80% or more, 85% or more, 90% or more, 95% or more, 96% or more, 97% or more, 98% or more, and 100% or less with the sequence of SEQ ID NO: 6, SEQ ID NO: 7, or SEQ ID NO: 8, but is not limited thereto.

Additionally, the polynucleotide of the present application may include a probe that may be prepared from a known gene sequence, for example, any sequence which can hybridize with a sequence complementary to all or part of the polynucleotide sequence of the present application under stringent conditions without limitation. The "stringent conditions" refers to conditions under which specific hybridization between polynucleotides is allowed. Such conditions are specifically described in the literature (J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York, 9.50-9.51, 11.7-11.8). For example, the stringent conditions may include conditions under which genes having a high homology or identity of 70% or more, 75% or more, 80% or more, 85% or more, 90% or more, 95% or more, 96% or more, 97% or more, 98% or more, or 99% or more are hybridized with each other and genes having a homology or identity lower than the above homologies or identities are not hybridized with each other, or washing conditions of Southern hybridization, that is, washing once, specifically twice or three times at a salt concentration and a temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present application may include isolated nucleotide fragments complementary to the entire sequence as well as nucleic acid sequences substantially similar thereto.

Specifically, polynucleotides having a homology or identity with the polynucleotide of the present application may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (e.g., Sambrook *et al.).*

In one embodiment of the present application, the nucleotide diphosphate to which glucose is bonded may be prepared by reacting sucrose and nucleotide diphosphate in the presence of a sucrose synthase, but is not limited thereto.

As used herein, the term "sucrose synthase" plays a role in the production of sucrose by reversibly transferring glucose, which is bonded to nucleotide diphosphate, to fructose in plant metabolism. In the present invention, the sucrose synthase demonstrates an activity to separate nucleotide diphosphate to which glucose is bonded and fructose by reacting sucrose and nucleotide diphosphate in a pH range of 5 to 10.

The sucrose synthase may be those derived from rice, corn, wheat, bamboo, *Arabidopsis thaliana,* grass, barley, sorghum or potato. Preferably, the sucrose synthase is those derived from rice, corn, wheat, or barley, and more preferably from rice, in particular, *Oryza sativa.* The sucrose synthase may be produced from recombinant *Escherichia coli, Bacillus,* yeast, *Corynebacterium* or *Agrobacterium* transformed with a vector containing a sucrose synthase gene, and may be further purified after it is produced from *Escherichia coli* and the like. The sucrose synthase may be those known in the art or may be commercially purchased, but is not limited thereto.

Specifically, the sucrose synthase of the present application may have and/or include an amino acid sequence of SEQ ID NO: 5 or may consist or essentially consist of the amino acid sequence.

The sucrose is not particularly limited so long as it can serve as a substrate for sucrose synthase to provide glucose to nucleotide diphosphate. Examples of sucrose may include raw sugar or sugar without limitation.

In the present application, purine nucleotide or pyrimidine nucleotide may be used as the nucleotide diphosphate. Preferably, uridine diphosphate is used as the nucleotide diphosphate, but the nucleotide diphosphate is not limited thereto.

The nucleotide diphosphate to which glucose is bonded may be reacted with rebaudioside A to prepare rebaudioside D by the uridine diphosphate (UDP)-glycosyltransferase B (UGT-B) of the present application.

In one embodiment of the present application, the rebaudioside A may be prepared by reacting nucleotide diphosphate to which glucose is bonded with stevioside in the presence of uridine diphosphate (UDP)-glycosyltransferase A (UGT-A), but is not limited thereto.

The uridine diphosphate (UDP)-glycosyltransferase A (UGT-A) may produce rebaudioside A by reacting the nucleotide diphosphate to which glucose is bonded with stevioside.

The uridine diphosphate (UDP)-glycosyltransferase A (UGT-A) may be those derived from *Oryza sativa, Stevia rebaudiana* Bertoni, *Bambusa oldhamii, Brachypodium distachyon, Hordeum vulgare, Sorghum bicolor,* Zea *mays,* or *Arabidopsis thaliana.* Preferably, the uridine diphosphate (UDP)-glycosyltransferase A (UGT-A) may be those derived from *Oryza sativa, Stevia rebaudiana* Bertoni, or *Bambusa oldhamii.* More preferably, it may be those derived from *Stevia rebaudiana* Bertoni. The uridine diphosphate (UDP)-glycosyltransferase A (UGT-A) may be those produced from recombinant *Escherichia coli, Bacillus,* yeast, *Corynebacterium* or *Agrobacterium* transformed with a vector containing a glycosyltransferase gene, or may be further purified after it is produced from *Escherichia coli* and the like. The uridine diphosphate (UDP)-glycosyltransferase A (UGT-A) may be those known in the art or may be commercially purchased, but is not limited thereto.

Specifically, the uridine diphosphate (UDP)-glycosyltransferase A (UGT-A) of the present application may have and/or include an amino acid sequence of SEQ ID NO: 4 or may consist or essentially consist of the amino acid sequence.

The stevioside is a hot water or aqueous ethanol solution extract from *Stevia rebaudiana* or purified material thereof, or a by-product after the production of rebaudioside A from the extract. Stevioside may be those having stevioside content of 10 wt% or more, preferably 50 wt% or more, particularly preferably 70 wt% or more, and more particular preferably 80 wt% or more, based on total weight of steviol glycoside, but is not limited thereto.

In one embodiment of the present application, the rebaudioside D may be prepared as illustrated in the Chemical Reaction 1 below, but is not limited thereto.

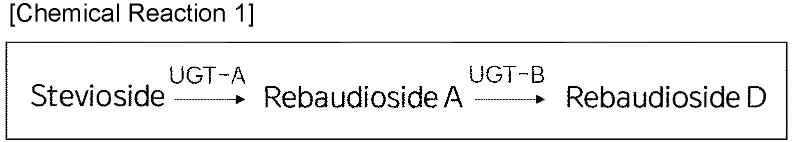

Specifically, the preparation method may be performed consecutively *in situ.*

As used herein, the term *"in situ"* means that a reaction is consecutively performed in a single reaction system.

The preparation method of the present application provides a consecutive reaction system, wherein one glucose is specifically bonded to the C-3' position of stevioside 13-O-glucose to synthesize rebaudioside A with high yield, and rebaudioside D is synthesized from rebaudioside A in accordance with the Chemical Reaction 1 above.

In one embodiment of the present application, the uridine diphosphate (UDP)-glycosyltransferase B (UGT-B) may be a protein consisting of an amino acid sequence of SEQ ID NO: 2, and rebaudioside D isomer may be further prepared, but is not limited thereto.

For example, the rebaudioside D may have the following structure, but is not limited thereto:

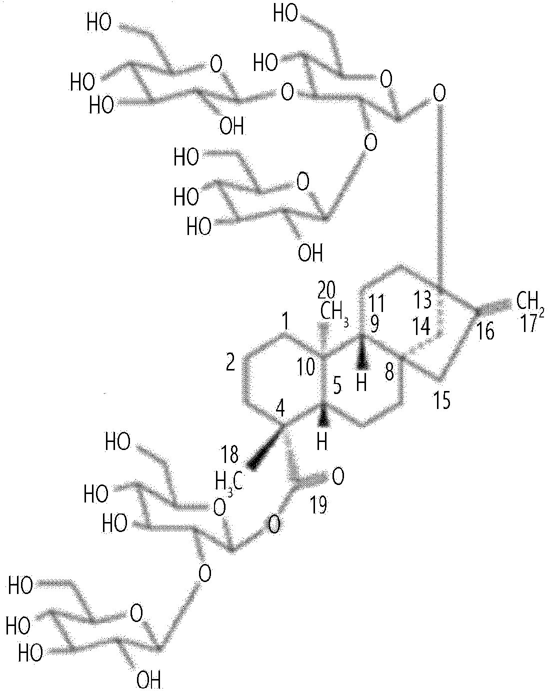

Another aspect of the present application provides a method for preparing rebaudioside M, including: reacting nucleotide diphosphate to which glucose is bonded with rebaudioside A in the presence of uridine diphosphate (UDP)-glycosyltransferase B (UGT-B) to prepare rebaudioside D; and
reacting the rebaudioside D with nucleotide diphosphate to which glucose is bonded in the presence of uridine diphosphate (UDP)-glycosyltransferase A (UGT-A) to prepare rebaudioside M,
wherein the uridine diphosphate (UDP)-glycosyltransferase B (UGT-B) is at least one protein selected from the group consisting of proteins consisting of an amino acid sequence of SEQ ID NOS: 1 to 3.

The nucleotide diphosphate, uridine diphosphate (UDP)-glycosyltransferase B (UGT-B), rebaudioside A, and rebaudioside D are as described above.

In one embodiment of the present application, the nucleotide diphosphate to which glucose is bonded may be prepared by reacting sucrose with nucleotide diphosphate in the presence of a sucrose synthase, as shown in the Chemical Reaction 1 above, but is not limited thereto.

Specifically, the sucrose synthase of the present application may have and/or include an amino acid sequence of SEQ ID NO: 5 or may consist or essentially consist of the amino acid sequence.

In one embodiment of the present application, the rebaudioside A may be prepared by reacting nucleotide diphosphate to which glucose is bonded with stevioside in the presence of uridine diphosphate (UDP)-glycosyltransferase A (UGT-A), as shown in the Chemical Reaction 1 above, but is not limited thereto.

Specifically, the uridine diphosphate (UDP)-glycosyltransferase A (UGT-A) of the present application may have and/or include an amino acid sequence of SEQ ID NO: 4 or may consist or essentially consist of the amino acid sequence.

The uridine diphosphate (UDP)-glycosyltransferase A (UGT-A) of the present application may produce rebaudioside M by reacting the nucleotide diphosphate to which glucose is bonded with rebaudioside D.

Specifically, the uridine diphosphate (UDP)-glycosyltransferase A (UGT-A) of the present application may have and/or include an amino acid sequence of SEQ ID NO: 4 or may consist or essentially consist of the amino acid sequence.

In one embodiment of the present application, the rebaudioside M may be prepared as illustrated in the Chemical Reaction 2 below, but is not limited thereto.

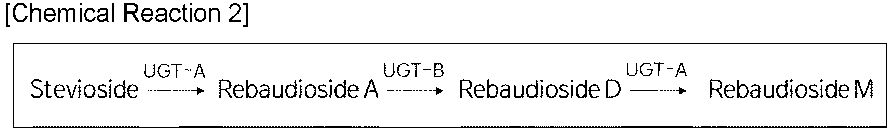

Specifically, the preparation method may be performed consecutively *in situ.*

The preparation method of the present application provides a consecutive reaction system, wherein rebaudioside A is synthesized from stevioside with high yield, rebaudioside D is synthesized from rebaudioside A, and rebaudioside M is synthesized from rebaudioside D, in accordance with the Chemical Reaction 2 above.

Accordingly, the preparation method of the present application uses raw materials such as stevioside and rebaudioside A, which are inexpensive and can be easily obtained, and can convert stevioside and rebaudioside A, which are bitter components contained in stevia extract, into rebaudioside D and rebaudioside M, which are components with excellent taste, and thus can be effectively used in the production of stevia sweetener with excellent sweetening quality.

In one embodiment of the present application, the uridine diphosphate (UDP)-glycosyltransferase B (UGT-B) may be a protein consisting of an amino acid sequence of SEQ ID NO: 2, and rebaudioside M isomer may be further prepared, but is not limited thereto.

For example, the rebaudioside M may have the following structure, but is not limited thereto:

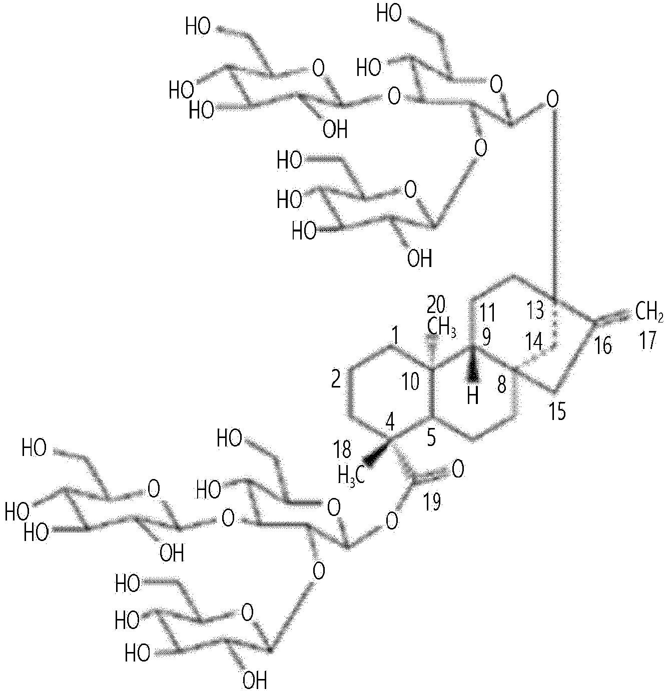

Still another aspect of the present application provides a method for preparing rebaudioside D from rebaudioside A, including: reacting sucrose, nucleotide diphosphate, rebaudioside A, sucrose synthase and uridine diphosphate (UDP)-glycosyltransferase B (UGT-B) *in situ* to prepare rebaudioside D,
wherein the uridine diphosphate (UDP)-glycosyltransferase B (UGT-B) is at least one protein selected from the group consisting of proteins consisting of an amino acid sequence of SEQ ID NOS: 1 to 3.

The sucrose, nucleotide diphosphate, rebaudioside A, sucrose synthase, uridine diphosphate (UDP)-glycosyltransferase B (UGT-B), rebaudioside D, and *in situ* are as described above.

In one embodiment of the present application, the uridine diphosphate (UDP)-glycosyltransferase B (UGT-B) may be a protein consisting of an amino acid sequence of SEQ ID NO: 2, and rebaudioside D isomer may be further prepared, but is not limited thereto.

Yet another aspect of the present application provides a method for preparing rebaudioside M from rebaudioside A, including: reacting sucrose, nucleotide diphosphate, rebaudioside A, rebaudioside D, sucrose synthase, uridine diphosphate (UDP)-glycosyltransferase A (UGT-A) and uridine diphosphate (UDP)-glycosyltransferase B (UGT-B) *in situ* to prepare rebaudioside M,
wherein the uridine diphosphate (UDP)-glycosyltransferase B (UGT-B) is at least one protein selected from the group consisting of proteins consisting of an amino acid sequence of SEQ ID NOS: 1 to 3.

The sucrose, nucleotide diphosphate, rebaudioside A, rebaudioside D, sucrose synthase, uridine diphosphate (UDP)-glycosyltransferase A (UGT-A), uridine diphosphate (UDP)-glycosyltransferase B (UGT-B), *in situ,* and rebaudioside M are as described above.

Specifically, the uridine diphosphate (UDP)-glycosyltransferase A (UGT-A) may be a protein consisting of an amino acid sequence of SEQ ID NO: 4, but is not limited thereto.

In one embodiment of the present application, the uridine diphosphate (UDP)-glycosyltransferase B (UGT-B) may be a protein consisting of an amino acid sequence of SEQ ID NO: 2, and rebaudioside M isomer may be further prepared, but is not limited thereto.

Even another aspect of the present application provides a composition for preparing rebaudioside D, including uridine diphosphate (UDP)-glycosyltransferase B (UGT-B), wherein the uridine diphosphate (UDP)-glycosyltransferase B (UGT-B) is at least one protein selected from the group consisting of proteins consisting of an amino acid sequence of SEQ ID NOS: 1 to 3.

The uridine diphosphate (UDP)-glycosyltransferase B (UGT-B) and rebaudioside D are as described above.

In one embodiment of the present application, the uridine diphosphate (UDP)-glycosyltransferase B (UGT-B) may be a protein consisting of an amino acid sequence of SEQ ID NO: 1, SEQ ID NO: 2, or SEQ ID NO: 3, and the rebaudioside D may be at least one selected from the group consisting of rebaudioside D and rebaudioside D isomer, but these are not limited thereto.

Further another aspect of the present application provides a composition for preparing rebaudioside M, including: uridine diphosphate (UDP)-glycosyltransferase A (UGT-A) and uridine diphosphate (UDP)-glycosyltransferase B (UGT-B), wherein the uridine diphosphate (UDP)-glycosyltransferase A (UGT-A) is a protein consisting of an amino acid sequence of SEQ ID NO: 4, and the uridine diphosphate (UDP)-glycosyltransferase B (UGT-B) is at least one protein selected from the group consisting of proteins consisting of an amino acid sequence of SEQ ID NOS: 1 to 3.

The uridine diphosphate (UDP)-glycosyltransferase A (UGT-A), uridine diphosphate (UDP)-glycosyltransferase B (UGT-B), and rebaudioside M are as described above.

In one embodiment of the present application, the uridine diphosphate (UDP)-glycosyltransferase B (UGT-B) may be a protein consisting of an amino acid sequence of SEQ ID NO: 1, SEQ ID NO: 2, or SEQ ID NO: 3, and the rebaudioside M may be at least one selected from the group consisting of rebaudioside M and rebaudioside M isomer, but these are not limited thereto.

The composition of the present application may further include any suitable excipient commonly used in the composition for producing amino acids, and such excipient may be, for example, a preservative, a wetting agent, a dispersing agent, a suspending agent, a buffering agent, a stabilizing agent, or an isotonic agent, but is not limited thereto.

Further another aspect of the present application provides a use of uridine diphosphate (UDP)-glycosyltransferase B (UGT-B) to convert rebaudioside A to rebaudioside D, wherein the uridine diphosphate (UDP)-glycosyltransferase B (UGT-B) is at least one protein selected from the group consisting of proteins consisting of an amino acid sequence of SEQ ID NOS: 1 to 3.

The uridine diphosphate (UDP)-glycosyltransferase A (UGT-A), uridine diphosphate (UDP)-glycosyltransferase B (UGT-B), rebaudioside A, rebaudioside M, *etc.* are as described above.

### [Mode for Carrying Out the Invention]

Hereinafter, the present application will be described in detail by way of Examples. However, these Examples are merely preferred Examples given for illustrative purposes, and thus, the scope of the present application is not intended to be limited to or by these Examples. Meanwhile, technical features which are not described herein can be sufficiently understood and easily carried out by those skilled in the art in the technical field of the present application or in a similar technical field.

### Example 1. Cultivation Conditions

A recombinant microorganism was prepared by introducing a polynucleotide encoding the uridine diphosphate (UDP)-glycosyltransferase B (UGT-B) of the present application, and then used in the reaction after expressing the uridine diphosphate (UDP)-glycosyltransferase B (UGT-B). The culture conditions of the recombinant **microorganism are as follows.**

### Example 1-1. Cultivation Conditions of E. coli

The recombinant *E*. *coli* was cultured as follows: a test tube containing 5 mL of LB medium containing kanamycin at a concentration of 50 µg/mL was inoculated with the recombinant *E*. *coli,* followed by seed culturing in an incubator at 37°C until the absorbance at 600 nm became 2.0. The seed cultured-solution was added to a flask containing 500 mL of LB medium containing kanamycin at a concentration of 50 µg/mL and then cultured. Further, 0.1 mM IPTG (isopropyl β-D-1-thiogalacthiopyranoside) was added until the absorbance at 600 nm became 0.4, thereby inducing mass expression of the enzymes. The culture conditions were adjusted so that the stirring speed was 180 rpm and the culture temperature was 37°C during the procedure, while the stirring speed was 120 rpm after the addition of IPTG.

### Example 1-2. Cultivation Conditions of Corynebacteria

The recombinant *Corynebacteria* were inoculated into a medium (Bacto-Trypton 10 g/L, Bacto-yeast extract 5 g/L, NaCl 5 g/L, Soytone 5 g/L) containing kanamycin at a concentration of 10 µg/mL with an initial concentration of O.D.₆₀₀ = 0.1, and cultured at 30°C for 24 hours to induce expression of the enzyme. The thus-obtained culture solution was inoculated into a fermenter containing a medium (glucose 80 g/L, soytone 20 g/L, (NH4)₂SO₄ 10 g/L, KH₂PO₄ 1.2 g/L, MgSO₄ 1.4 g/L) containing kanamycin at a concentration of 10 µg/mL with O.D.₆₀₀ = 0.6 and cultured at 30°C for 24 hours.

### Example 2. Measurement of Enzymatic Activity of Uridine Diphosphate (UDP)-Glycosyltransferase B (UGT-B) for Rebaudioside A as Raw Material

### Example 2-1. Purification of Uridine Diphosphate (UDP)-Glycosyltransferase B (UGT-B)

For the uridine diphosphate (UDP)-glycosyltransferase B (UGT-B) (UGT-B; SEQ ID NO: 1, SEQ ID NO: 2, or SEQ ID NO: 3) of the present application that converts rebaudioside A to rebaudioside D, each recombinant plasmid (vector-pET28a) containing a gene (SEQ ID NO: 6, SEQ ID NO: 7, or SEQ ID NO: 8) encoding uridine diphosphate (UDP)-glycosyltransferase B (UGT-B) was prepared and cloned into E. *coli* BL21 (DE3) to induce mass expression of each enzyme, and then the enzymes were purified and used.

Specifically, a test tube containing 5 mL of LB medium was inoculated with the recombinant strain BL21 (DE3), followed by seed culturing in an incubator at 37°C until the absorbance at 600 nm became 2.0. The seed cultured-solution was added to a flask containing 500 mL of LB medium and then cultured. Further, 0.1 mM IPTG (isopropyl β-D-1-thiogalacthiopyranoside) was added until the absorbance at 600 nm became 0.4, thereby inducing mass expression of the enzymes. The culture conditions were adjusted so that the stirring speed was 180 rpm and the culture temperature was 37°C during the procedure, while the stirring speed was 120 rpm and the culture temperature was 16°C after the addition of IPTG. The culture solution of the transformed strain was centrifuged at 6,000 g at 4°C for 20 minutes to separate cell supernatant as an enzyme solution. In order to exactly identify the properties of the enzymes, the enzyme solution was purified using a Ni-NTA superflow column.

Each enzyme mass-expressed using a recombinant plasmid containing SEQ ID NO: 6, SEQ ID NO: 7, or SEQ ID NO: 8 was named UGT-B_6, UGT-B_7, and UGT-B_8, respectively.

Meanwhile, the recombinant *Corynebacteria* were inoculated into a medium (Bacto-Trypton 10 g/L, Bacto-yeast extract 5 g/L, NaCl 5 g/L, Soytone 5 g/L) containing kanamycin at a concentration of 10 µg/mL with an initial concentration of O.D.₆₀₀ = 0.1, and cultured at 30°C for 24 hours to induce expression of the enzyme. The thus-obtained culture solution was inoculated into a fermenter containing a medium (glucose 80 g/L, soytone 20 g/L, (NH₄)₂SO₄ 10 g/L, KH₂PO₄ 1.2 g/L, MgSO₄ 1.4 g/L) containing kanamycin at a concentration of 10 µg/mL with O.D.₆₀₀ = 0.6 and cultured at 30°C for 24 hours.

### Example 2-2. Measurement of Enzymatic Activity of Uridine Diphosphate (UDP)-Glycosyltransferase B (UGT-B) for Rebaudioside A as Raw Material

The enzymatic activity of uridine diphosphate (UDP)-glycosyltransferase B (UGT-B) for rebaudioside A as raw material was measured using each of the enzymes prepared in Example 2-1.

Specifically, the raw material used in the enzymatic reaction was RebA (Daepyeong), which was dissolved in water to a concentration of 2 mM. The reaction was carried out at 37°C for 16 hours using each of the enzymes that were expressed in microorganisms and prepared in Example 2-1, and then analyzed by HPLC. The raw material aqueous solution may contain UDP-glucose or UDP(Uridine-diphosphate) at 2 mM.

The conditions for HPLC Analysis are as follows:
- Detector wavelength: 210 nm
- Flow rate: 1 mL/min
- Sample injection vol.: 10 µL
- Column: Capcell pak C18 MG II (Shiseido, 250 mm × 4.6 mm, particle size: 5 µm)
- Solvent: Acetonitrile 30%

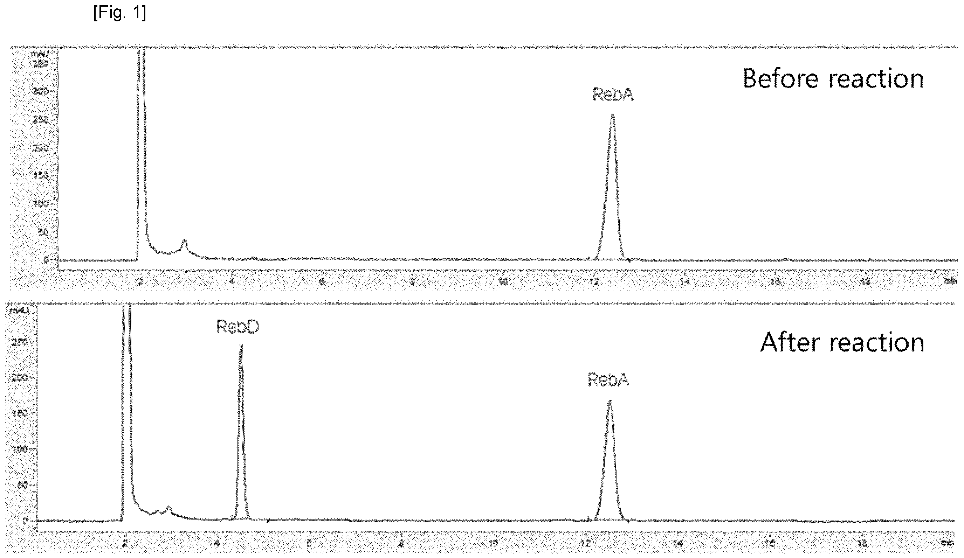

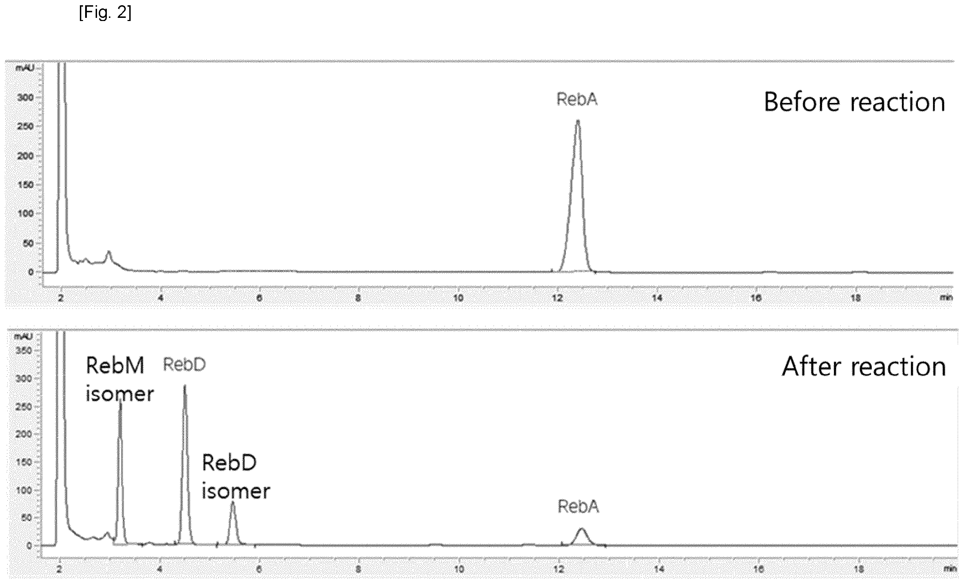

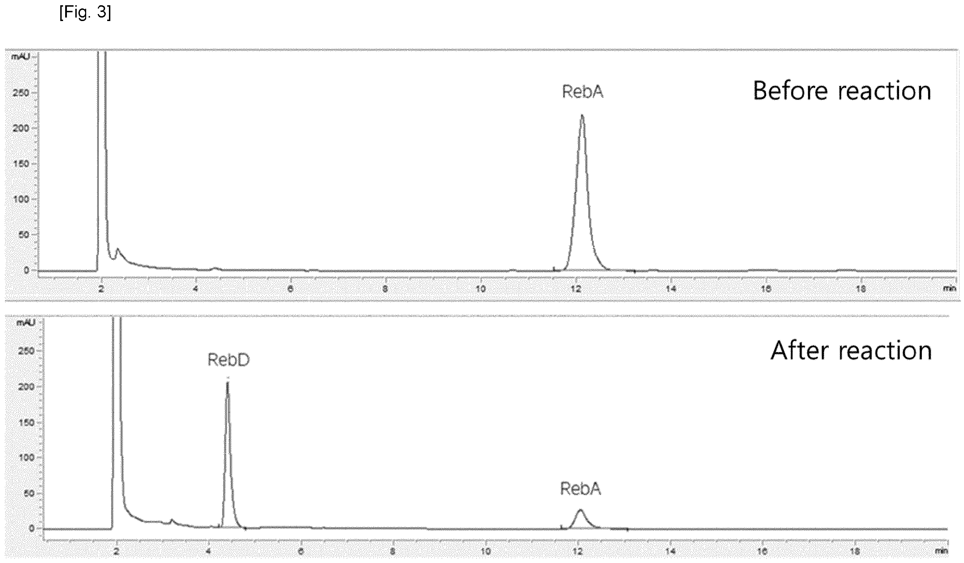

The measurement results are shown in Table 1 below and Figs. 1 to 3.

**[Table 1]**

| **Sample** | **Rebaudioside M Isomer Content (%)** | **Rebaudioside D Isomer Content (%)** | **Rebaudioside D Content (%)** | **Rebaudioside A Content (%)** |
|---|---|---|---|---|
| Raw Material (Reaction Time: 0) | | | 0.3 | 99.7 |
| UGT-B_6 | | | 39.5 | 60.5 |
| UGT-B_7 | 31.5 | 14.1 | 44.0 | 10.4 |
| UGT-B_8 | | | 76.1 | 23.9 |

As shown in Table 1 above and Figs. 1 to 3, it was confirmed that rebaudioside A was converted to rebaudioside D by the uridine diphosphate (UDP)-glycosyltransferases B (UGT-B_6 and UGT-B_8) of the present application, and that rebaudioside A was converted to rebaudioside M isomer, rebaudioside D isomer, and rebaudioside D by the uridine diphosphate (UDP)-glycosyltransferase B (UGT-B_7).

### Example 3. Measurement of Enzymatic Activity of Uridine Diphosphate (UDP)-Glycosyltransferase A (UGT-A) and Uridine Diphosphate (UDP)-Glycosyltransferase B (UGT-B) for Rebaudioside A as Raw Material

The uridine diphosphate (UDP)-glycosyltransferase A (UGT-A) and sucrose synthetase were purified using the method disclosed in the prior patent literature (WO 2014/133248), and their sequences were shown in SEQ ID NO: 4 and SEQ ID NO: 5, respectively.

The enzymatic activity of uridine diphosphate (UDP)-glycosyltransferase A (UGT-A) and uridine diphosphate (UDP)-glycosyltransferase B (UGT-B) for rebaudioside A as raw material was measured using the uridine diphosphate (UDP)-glycosyltransferase A (UGT-A), the sucrose synthetase, and each of the uridine diphosphate (UDP)-glycosyltransferases B (UGT-B) prepared in Example 2-1.

Specifically, the raw material used for the enzymatic reaction were 2 mM stevioside (Haigen) and a 50 mM sugar (CJ Cheiljedang) aqueous solution. The reaction was carried out at 37°C for 24 hours using the uridine diphosphate (UDP)-glycosyltransferase A (UGT-A), the sucrose synthetase, and each of the uridine diphosphate (UDP)-glycosyltransferases B (UGT-B) prepared in Example 2-1, which were expressed in microorganisms, and then analyzed by HPLC. The raw material aqueous solution may contain UDP-glucose or UDP (uridine diphosphate) at a concentration of 2 mM.

The conditions for HPLC Analysis are as follows:
- Detector wavelength: 210 nm
- Flow rate: 1 mL/min
- Sample injection vol.: 10 µL
- Column: Capcell pak C18 MG II (Shiseido, 250 mm × 4.6 mm, particle size: 5 µm)
- Solvent: Acetonitrile 30%

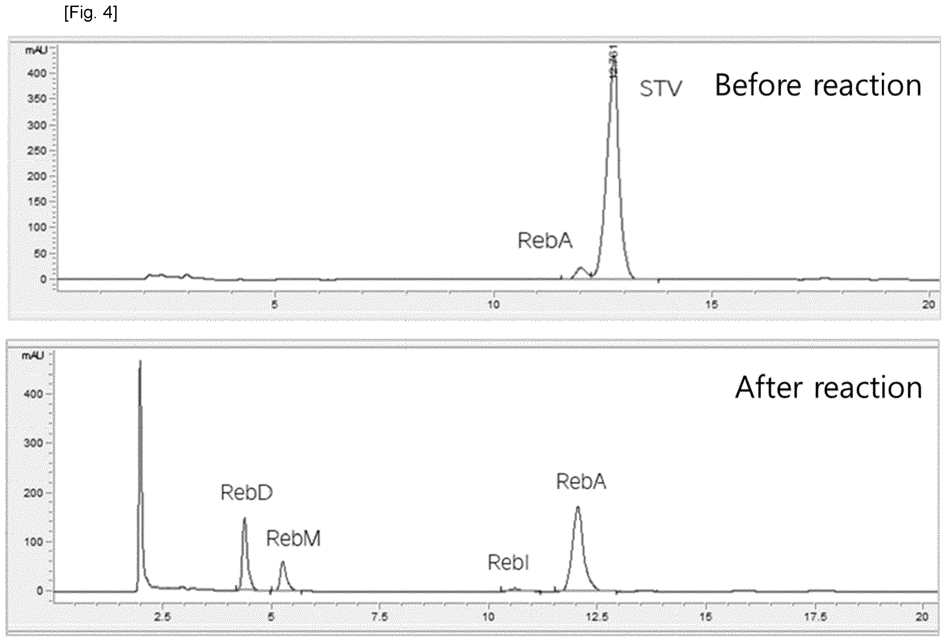

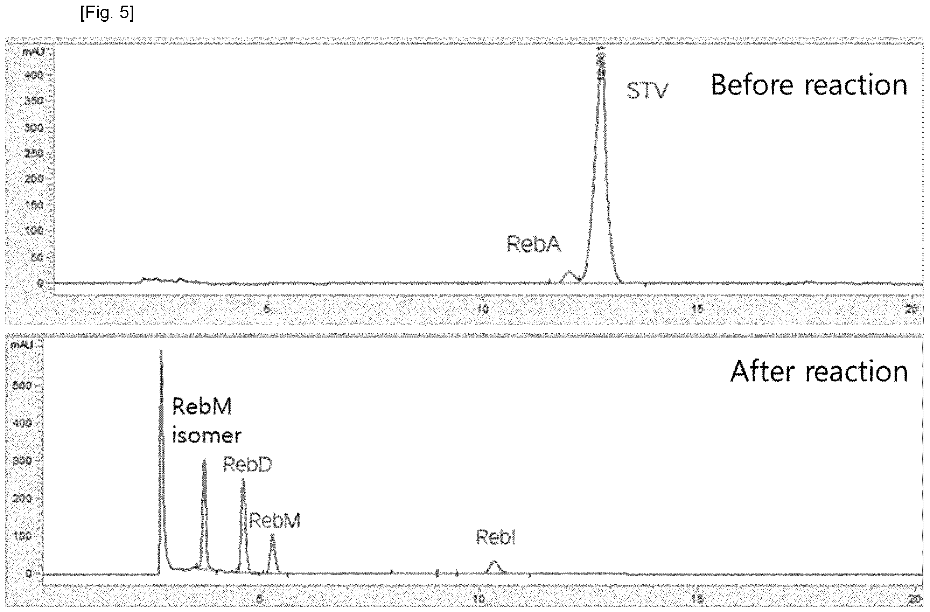

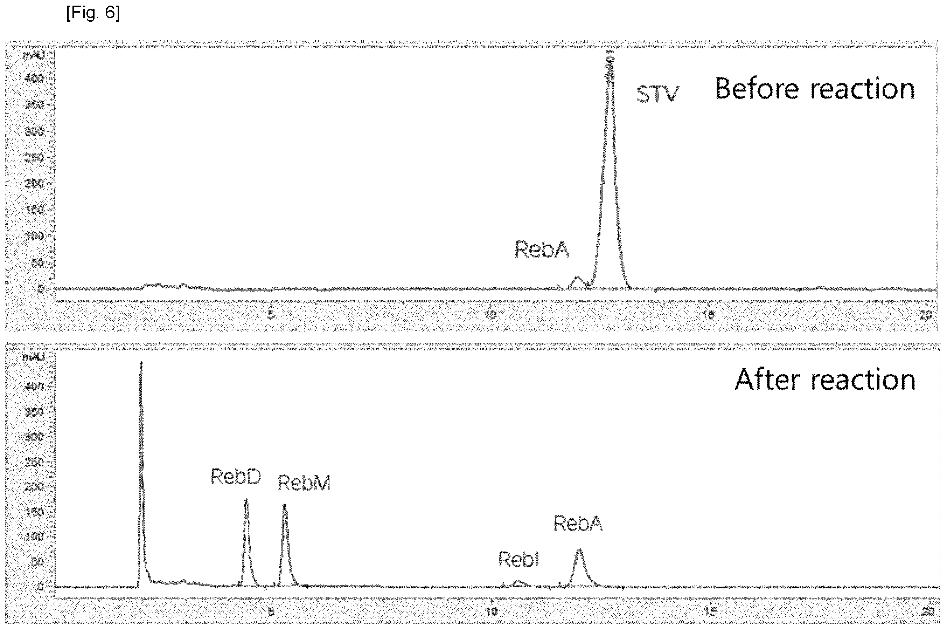

The measurement results are shown in Table 2 below and Figs. 4 to 6.

**[Table 2]**

| **Sample** | **Rebaudio side M Isomer (%)** | **Rebaudio side D (%)** | **Rebaudio side M (%)** | **Rebaudio side I (%)** | **Rebaudio side A (%)** | **Stevios ide (%)** |
|---|---|---|---|---|---|---|
| Raw Material (Reacti on Time: 0) | 0 | 0 | 0 | 0 | 4.6 | 95.4 |
| UGT-B_6, | 0 | 24.3 | 12.1 | 1.8 | 61.8 | 0 |
| UGT-A and sucrose synthet ase | | | | | | |
| UGT-B_7, UGT-A and sucrose synthet ase | 37.5 | 35 | 16.7 | 10.8 | 0 | 0 |
| UGT-B_8, UGT-A and sucrose synthet ase | 0 | 31.0 | 34.3 | 4.7 | 30 | 0 |

As shown in Table 2 above and Figs. 4 to 6, it was confirmed that 100% of stevioside was converted to rebaudioside M isomer, rebaudioside D, rebaudioside M and rebaudioside I, and rebaudioside A relative to the molar concentration.

### Example 4. Measurement of Enzymatic Activity of Uridine Diphosphate (UDP)-Glycosyltransferase A (UGT-A) for Rebaudioside D as Raw Material

The conversion rate of rebaudioside M from rebaudioside D was measured by the uridine diphosphate (UDP)-glycosyltransferase A (UGT-A) of Example 3.

Specifically, the raw material used for the enzymatic reaction was rebaudioside D (Haigen), which was dissolved in water to a concentration of 1 mM. The reaction was carried out at 37°C for 16 hours using the uridine diphosphate (UDP)-glycosyltransferase A (UGT-A) expressed in microorganisms, and then analyzed by HPLC. The raw material aqueous solution may contain UDP-glucose or UDP (uridine diphosphate) at a concentration of 2 mM.

The conditions for HPLC Analysis are as follows:
- Detector wavelength: 210 nm
- Flow rate: 1 mL/min
- Sample injection vol.: 10 µL
- Column: Capcell pak C18 MG II (Shiseido, 250 mm × 4.6 mm, particle size: 5 µm)
- Solvent: Acetonitrile 30%

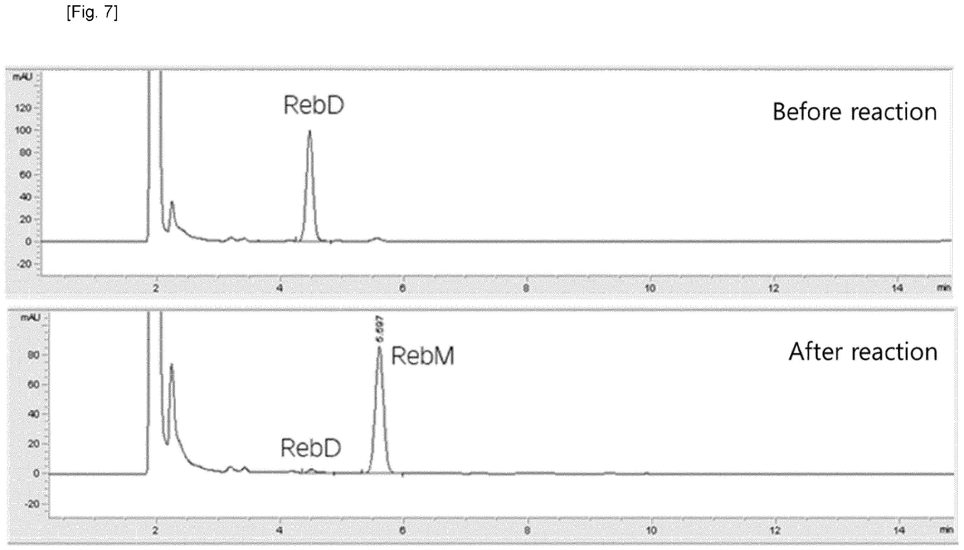

The measurement results are shown in Fig. 7.

As shown in Fig. 7, it was confirmed that most of rebaudioside D was converted to rebaudioside M by the uridine diphosphate (UDP)-glycosyltransferase A (UGT-A) of the present application.

### Example 5. Liquid Chromatography Mass Spectrometry (LC-MS/MS) Analysis of Rebaudioside D Isomer and Rebaudioside M Isomer

In order to confirm whether the compound converted in Examples 2 and 3 is a rebaudioside D isomer or a rebaudioside M isomer, the rebaudioside D isomer and the rebaudioside M isomer converted in Examples 2 and 3 were compared with rebaudioside D and rebaudioside M, respectively, using liquid chromatography mass spectrometry (LC-MS/MS) analysis.

Specifically, Acquity UPLC (Waters) and Xe-vo G2-XS Q-Tof mass spectrometer were used. LC-MS/MS analysis conditions are shown in Table 3 below, elution conditions are shown in Table 4 below, and MS conditions are shown in Table 5 below.

**[Table 3]**

| LC-MS/MS Analysis Conditions | |
|---|---|
| Chromatography | Waters Acquity UPLC |
| Column | Waters Acquity UPLC BEH C18 1.7 µm 2.1 mm × |
| | 150 mm |
| Column temperature | 40°C |
| Flow rate | 0.20 mL/min |
| Sample injection | 1.0 µL |
| Solvent | A: Distilled water |
| | B: 50% Acetonitrile |

**[Table 4]**

| Elution Conditions | | |
|---|---|---|
| Time (min) | %A | %B |
| 0 | 55.0 | 45.0 |
| 0.50 | 55.0 | 45.0 |
| 3.00 | 52.5 | 47.5 |
| 6.00 | 52.4 | 47.6 |
| 9.00 | 47.5 | 52.5 |
| 9.10 | 0.0 | 100.0 |
| 10.00 | 0.0 | 100.0 |
| 10.10 | 55.0 | 45.0 |
| 15.00 | 55.0 | 45.0 |

**[Table 5]**

| MS conditions | |
|---|---|
| Ionization mode | ESI Negative mode |
| Capillary voltage | 2.5 kV |
| Cone voltage | 30 V |
| Source Temperature | 120°C |

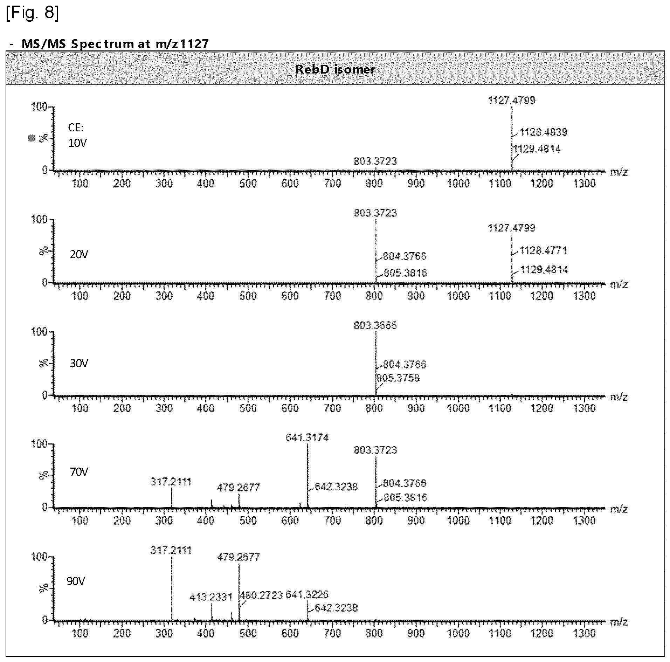

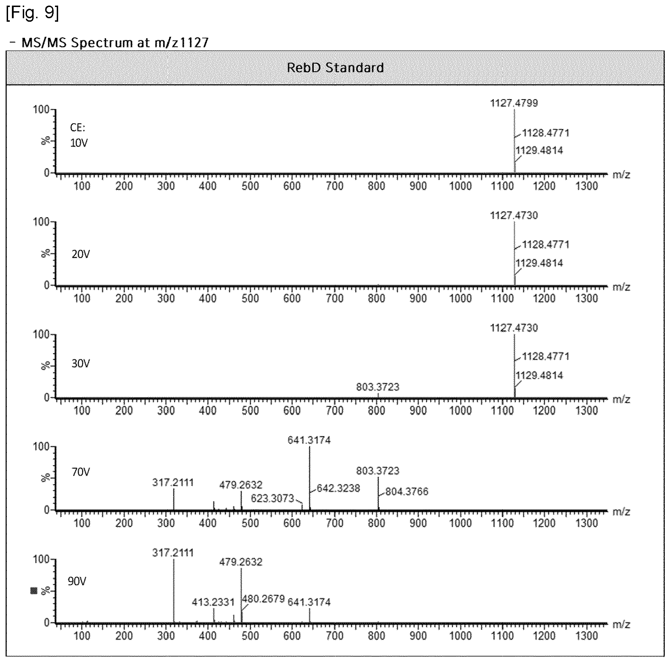

As a result, as shown in Figs. 8 and 9, the comparison of RebD Standard and Unknown Peak 1 revealed that both RebD Standard and Unknown Peak 1 have the same molecular weight, and their MSMS Fragments match with each other but differ only in the retention time of the peak, thus confirming that Unknown Peak 1 is an isomer of RebD.

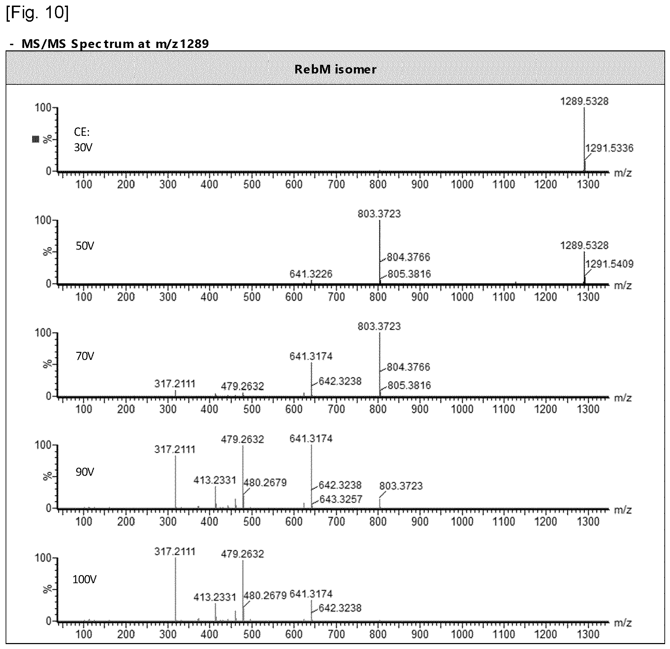

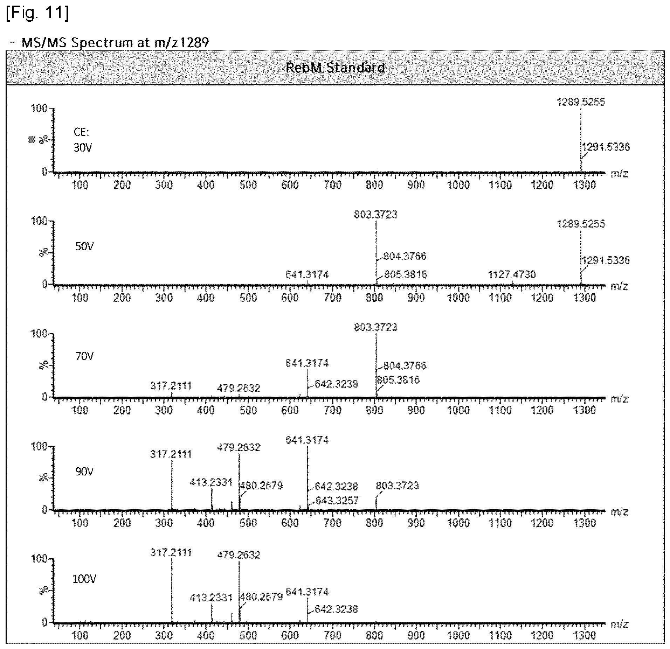

In addition, as shown in Figs. 10 and 11, the comparison of RebM Standard and Unknown Peak 2 revealed that both RebM Standard and Unknown Peak 2 have the same molecular weight, and their MSMS Fragments match with each other but differ only in the retention time of the peak, thus confirming that Unknown Peak 2 is an isomer of RebM.

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. The scope of the present application is therefore indicated by the appended claims rather than by the foregoing description. All changes which come within the meaning and range of equivalency of the claims are to be embraced within the scope of the present application.

## Claims

1. A method for preparing rebaudioside D, comprising:
reacting nucleotide diphosphate to which glucose is bonded with rebaudioside A in the presence of uridine diphosphate (UDP)-glycosyltransferase B (UGT-B) to prepare rebaudioside D,
wherein the uridine diphosphate (UDP)-glycosyltransferase B (UGT-B) is at least one protein selected from the group consisting of proteins consisting of an amino acid sequence of SEQ ID NOS: 1 to 3.

2. The method of claim 1, wherein the nucleotide diphosphate to which glucose is bonded is prepared by reacting sucrose and nucleotide diphosphate in the presence of a sucrose synthase.

3. The method of claim 1, wherein the rebaudioside A is prepared by reacting nucleotide diphosphate to which glucose is bonded with stevioside in the presence of uridine diphosphate (UDP)-glycosyltransferase A (UGT-A).

4. The method of claim 2, wherein the sucrose synthase is a protein consisting of an amino acid sequence of SEQ ID NO: 5.

5. The method of claim 3, wherein the uridine diphosphate (UDP)-glycosyltransferase A (UGT-A) is a protein consisting of an amino acid sequence of SEQ ID NO: 4.

6. The method of claim 1, wherein the uridine diphosphate (UDP)-glycosyltransferase B (UGT-B) is a protein consisting of an amino acid sequence of SEQ ID NO: 2, and rebaudioside D isomer is further prepared.

7. The method of any one of claims 1 to 6, wherein the method is performed consecutively *in situ.*

8. A method for preparing rebaudioside M, comprising:
reacting nucleotide diphosphate to which glucose is bonded with rebaudioside A in the presence of uridine diphosphate (UDP)-glycosyltransferase B (UGT-B) to prepare rebaudioside D; and
reacting the rebaudioside D with nucleotide diphosphate to which glucose is bonded in the presence of uridine diphosphate (UDP)-glycosyltransferase A (UGT-A) to prepare rebaudioside M,
wherein the uridine diphosphate (UDP)-glycosyltransferase B (UGT-B) is at least one protein selected from the group consisting of proteins consisting of an amino acid sequence of SEQ ID NOS: 1 to 3.

9. The method of claim 8, wherein the uridine diphosphate (UDP)-glycosyltransferase A (UGT-A) is a protein consisting of an amino acid sequence of SEQ ID NO: 4.

10. The method of claim 8, wherein the nucleotide diphosphate to which glucose is bonded is prepared by reacting sucrose and nucleotide diphosphate in the presence of a sucrose synthase.

11. The method of claim 8, wherein the rebaudioside A is prepared by reacting nucleotide diphosphate to which glucose is bonded with stevioside in the presence of uridine diphosphate (UDP)-glycosyltransferase A (UGT-A).

12. The method of claim 10, wherein the sucrose synthase is a protein consisting of an amino acid sequence of SEQ ID NO: 5.

13. The method of claim 11, wherein the uridine diphosphate (UDP)-glycosyltransferase A (UGT-A) is a protein consisting of an amino acid sequence of SEQ ID NO: 4.

14. The method of claim 8, wherein the uridine diphosphate (UDP)-glycosyltransferase B (UGT-B) is a protein consisting of an amino acid sequence of SEQ ID NO: 2, and rebaudioside M isomer is further prepared.

15. The method of any one of claims 8 to 14, wherein the method is performed consecutively *in situ.*

16. A method for preparing rebaudioside D from rebaudioside A, comprising:
reacting sucrose, nucleotide diphosphate, rebaudioside A, sucrose synthase and uridine diphosphate (UDP)-glycosyltransferase B (UGT-B) *in situ* to prepare rebaudioside D,
wherein the uridine diphosphate (UDP)-glycosyltransferase B (UGT-B) is at least one protein selected from the group consisting of proteins consisting of an amino acid sequence of SEQ ID NOS: 1 to 3.

17. The method of claim 16, wherein the uridine diphosphate (UDP)-glycosyltransferase B (UGT-B) is a protein consisting of an amino acid sequence of SEQ ID NO: 2, and rebaudioside D isomer is further prepared.

18. A method for preparing rebaudioside M from rebaudioside A, comprising:
reacting sucrose, nucleotide diphosphate, rebaudioside A, rebaudioside D, sucrose synthase, uridine diphosphate (UDP)-glycosyltransferase A (UGT-A) and uridine diphosphate (UDP)-glycosyltransferase B (UGT-B) *in situ* to prepare rebaudioside M,
wherein the uridine diphosphate (UDP)-glycosyltransferase B (UGT-B) is at least one protein selected from the group consisting of proteins consisting of an amino acid sequence of SEQ ID NOS: 1 to 3.

19. The method of claim 18, wherein the uridine diphosphate (UDP)-glycosyltransferase A (UGT-A) is a protein consisting of an amino acid sequence of SEQ ID NO: 4.

20. The method of claim 18, wherein the uridine diphosphate (UDP)-glycosyltransferase B (UGT-B) is a protein consisting of an amino acid sequence of SEQ ID NO: 2, and rebaudioside M isomer is further prepared.

21. A composition for preparing rebaudioside D, comprising uridine diphosphate (UDP)-glycosyltransferase B (UGT-B), which is at least one protein selected from the group consisting of proteins consisting of an amino acid sequence of SEQ ID NOS: 1 to 3.

22. The composition of claim 21, wherein the uridine diphosphate (UDP)-glycosyltransferase B (UGT-B) is a protein consisting of an amino acid sequence of SEQ ID NO: 2, and the rebaudioside D is at least one selected from the group consisting of rebaudioside D and rebaudioside D isomer.

23. A composition for preparing rebaudioside M, comprising: uridine diphosphate (UDP)-glycosyltransferase B (UGT-B), which is at least one protein selected from the group consisting of proteins consisting of an amino acid sequence of SEQ ID NOS: 1 to 3, and uridine diphosphate (UDP)-glycosyltransferase A (UGT-A), which is a protein consisting of an amino acid sequence of SEQ ID NO: 4.

24. The composition of claim 23, wherein the uridine diphosphate (UDP)-glycosyltransferase B (UGT-B) is a protein consisting of an amino acid sequence of SEQ ID NO: 2, and the rebaudioside M is at least one selected from the group consisting of rebaudioside M and rebaudioside M isomer.
